# EUROPEAN PATENT APPLICATION

(11) **EP 1 748 082 A1**
(43) Date of publication of application: **31.01.2007**
(21) Application number: 05736968.8
(22) Date of filing: 27.04.2005
(51) Int. Cl.: C12Q 1/68, C12N 15/09, G01N 21/78, G01N 33/53, G01N 33/543, G01N 33/566, G01N 33/58, G01N 37/00

(54) **METHOD OF ANALYZING NUCLEIC ACID**

(30) Priority: 30.04.2004 JP 2004136643
(71) Applicant: Olympus Corporation, Tokyo 151-0072 (JP)
(72) Inventor: NAGAOKA, Tomonori, Tokyo 1660001 (JP)
(74) Representative: Wibbelmann, Jobst
(86) International application number: PCT/JP2005/007992
(87) International publication number: WO 2005/106029

(57) **Abstract**

An object of the invention is to avoid any possibility of error caused by a nucleic acid used as a standard, a probe, or probe spot, etc. or enable correction thereof, to analyze the frequency of gene expressions with high reproducibility, and to analyze any change of primary structure in a sample nucleic acid. The invention provides a method of analyzing the primary structure of at least one target base sequence present in a sample nucleic acid by detecting any signal from a sample nucleic acid hybrid composed of a probe and the same nucleic acid, wherein any signal from a synthetic standard nucleic acid hybrid composed of the probe and a synthetic standard nucleic acid containing at least one target base sequence is detected and on the basis of the signal from the synthetic standard nucleic acid hybrid, the signal from the sample nucleic acid hybrid is corrected to thereby effect analysis of the primary structure of the sample nucleic acid.

### [Sequence Table]

SEQUENCE LISTING

<110> OLYMPUS CORPORATION
<120> Method for analyzing nucleic acid
<130> PC9562
<160> 19

<210> 1
<211> 40
<212> DNA
<213> Artificial sequence
<220>
<223> Synthetic DNA probe
<400> 1
tgcctaggcg ttctactttg agtttggatg gtcctaacat 40

<210> 2
<211> 40
<212> DNA
<213> Artificial sequence
<220>
<223> Synthetic DNA probe
<400> 2
atgttaggac catccaaact caaagtagaa cgcctaggca 40

<210> 3
<211> 40
<212> DNA
<213> Artificial sequence
<220>
<223> Synthetic DNA probe
<400> 3
cgtgagccac agcggccatc ctgaaaccat tctatatgaa 40

<210> 4
<211> 40
<212> DNA
<213> Artificial sequence
<220>
<223> Synthetic DNA probe
<400> 4
ttcatataga atggtttcag gatggccgct gtggctcacg 40

<210> 5
<211> 40
<212> DNA
<213> Artificial sequence
<220>
<223> Synthetic DNA probe
<400> 5
agataggggt tcaagactga ttcctgcagc cactgttaag 40

<210> 6
<211> 40
<212> DNA
<213> Artificial sequence
<220>
<223> Synthetic DNA probe
<400> 6
cttaacagtg gctgcaggaa tcagtcttga acccctatct 40

<210> 7
<211> 40
<212> DNA

<213> Artificial sequence
<220>
<223> Synthetic DNA probe
<400> 7
ccggaaatgt gatcagaggt ggatccctag tagtatggaa 40

<210> 8
<211> 40
<212> DNA
<213> Artificial sequence
<220>
<223> Synthetic DNA probe
<400> 8
ttccatacta ctagggatcc acctctgatc acatttccgg 40

<210> 9
<211> 40
<212> DNA
<213> Artificial sequence
<220>
<223> Synthetic DNA probe
<400> 9
tataaacttg aggtagaagg agctggtggg gtaggcaaga 40

<210> 10
<211> 40
<212> DNA
<213> Artificial sequence
<220>
<223> Synthetic DNA probe
<400> 10
tcttgcctac cccaccagct ccttctacct caagtttata 40

<210> 11
<211> 39
<212> DNA
<213> Artificial sequence
<220>
<223> Synthetic DNA probe
<400> 11
gcaaatagag aaagaaaggg gtccccagtc ctcatgtac 39

<210> 12
<211> 39
<212> DNA
<213> Artificial sequence
<220>
<223> Synthetic DNA probe
<400> 12
gtacatgagg actggggacc cctttctttc tctatttgc 39

<210> 13
<211> 35
<212> DNA
<213> Artificial sequence
<220>
<223> grlAB:356U35
<400> 13
caaggtggctataaaacttcaggtggtcttcacgg 35

<210> 14
<211> 35
<212> DNA
<213> Artificial sequence

<220>
<223> grlAB:356U35
<400> 14
ccgtgaagaccacctgaagttttatagccaccttg 35

<210> 15
<211> 37
<212> DNA
<213> Artificial sequence
<220>
<223> grlAB:2550-37
<400> 15
aggttacgcgacagatataccaccacataatttagct 37

<210> 16
<211> 37
<212> DNA
<213> Artificial sequence
<220>
<223> grlAB:2550-37
<400> 16
agctaaattatgtggtggtatatctgtcgcgtaacct 37

<210> 17
<211> 4565
<212> DNA
<213> Artificial sequence
<220>
<223> Insert sequence
<400> 17
gaattccgac gtacgtttgc aggaggcgaa atcattggca atgaataaac aaaataatta
ttcagatgat tcaatacagg ttttagaggg gttagaagca gttcgtaaaa gacctggtat
gtatattgga tcaactgata aacggggatt acatcatcta gtatatgaaa ttgtcgataa
ctccgtcgat gaagtattga atggttacgg taacgaaata gatgtaacaa ttaataaaga
tggtagtatt tctatagaag ataatggacg tggtatgcca acaggtatac ataaatcagg
taaaccgaca gtcgaagtta tctttactgt tttacatgca ggaggtaaat ttggacaagg
tggctataaa acttcaggtg gtcttcacgg cgttggtgct tcagtggtaa atgcattgag
tgaatggctt gaagttgaaa tccatcgaga tggtaatata tatcatcaaa gttttaaaaa
cggtggttcg ccatcttcag gtttagtgaa aaaaggtaaa actaagaaaa caggtaccaa
agtaacattt aaacctgatg acacaatttt taaagcatct acatcattta attttgatgt
tttaagtgaa cgactacaag agtctgcgtt cttattgaaa aatttaaaaa taacgcttaa
tgatttacgc agtggtaaag agcgtcaaga gcattaccat tatgaagaag gaatcaaaga
gtttgttagt tatgtcaatg aaggaaaaga agttttgcat gacgtggcta cattttcagg
tgaagcaaat ggtatagagg tagacgtagc tttccaatat aatgatcaat attcagaaag
tattttaagt tttgtaaata atgtacgtac taaagatggt ggtacacatg aagttggttt
taaaacagca atgacacgcg tatttaatga ttatgcacgt cgtattaatg aacttaaaac
aaaagataaa aacttagatg gtaatgatat tcgtgaaggt ttaacagctg ttgtgtctgt
tcgtattcca gaagaattat tgcaatttga aggacaaacg aaatctaaat tgggtacttc
tgaagctaga agtgctgttg attcagttgt tgcagacaaa ttgccattct atttagaaga
aaaaggacaa ttgtctaaat cacttgtgaa aaaagcgatt aaagcacaac aagcaaggga
agctgcacgt aaagctcgtg aagatgctcg ttcaggtaag aaaaacaagc gtaaagacac
tttgctatct ggtaaattaa cacctgcaca aagtaaaaac actgaaaaaa atgaattgta
tttagtcgaa ggtgattctg cgggaggttc agcaaaactt ggacgagacc gcaaattcca
agcgatatta ccattacgtg gtaaggtaat taatacagag aaagcacgtc tagaagatat
ttttaaaaat gaagaaatta atacaattat ccacacaatc ggggcaggcg ttggtactga
ctttaaaatt gaagatagta attataatcg tgtaattatt atgactgatg ctgatactga
tggtgcgcat attcaagtgc tattgttaac attcttcttc aaatatatga aaccgcttgt
tcaagcaggt cgtgtattta ttgctttacc tccactttat aaattggaaa aaggtaaagg
caaaacaaag cgagttgaat acgcttggac agacgaagag cttaataaat tgcaaaaaga
acttggtaaa ggcttcacgt tacaacgtta caaaggtttg ggtgaaatga accctgaaca
attatgggaa acgacgatga acccagaaac acgaacttta attcgtgtac aagttgaaga
tgaagtgcgt tcatctaaac gtgtaacaac attaatgggt gacaaagtac aacctagacg
tgaatggatt gaaaagcatg ttgagtttgg tatgcaagag gaccaaagta ttttagataa
ttctgaagta caagtgcttg aaaatgatca atttgatgag gaggaaatct agtgagtgaa

ataattcaag atttatcact tgaagatgtt ttaggtgatc gctttggaag atatagtaaa
tatattattc aagagcgtgc attgccagat gttcgtgatg gtttaaaacc agtacaacgt
cgtattttat acgcaatgta ttcaagtggt aatacacacg ataaaaattt ccgtaaaagt
gcgaaaacag tcggtgatgt tattggtcaa tatcatccac atggagactc ctcagtgtac
gaagcaatgg tccgtttaag tcaagactgg aagttacgac atgtcttaat agaaatgcat
ggtaataatg gtagtatcga taatgatccg ccagcggcaa tgcgttacac tgaagctaag
ttaagcttac tagctgaaga gttattacgt gatattaata aagagacagt ttctttcatt
ccaaactatg atgatacgac actcgaacca atggtattgc catcaagatt tcctaactta
ctagtgaatg gttctacagg tatatctgca ggttacgcga cagatatacc accacataat
ttagctgaag tgattcaagc aacacttaaa tatattgata atccggatat tacagtcaat
caattaatga aatatattaa aggtcctgat tttccaactg gtggtattat tcaaggtatt
gatggtatta aaaaagctta tgaatcaggt aaaggtagaa ttatagttcg ttctaaagtt
gaagaagaaa ctttacgcaa tggacgtaaa cagttaatta ttactgaaat tccatatgaa
gtgaacaaag gtagcttagt aaaacgtatc gatgaattac gtgctgacaa aaaagtcgat
ggtatcgttg aagtacgtga tgaaactgat agaactggtt tacgaatagc aattgaattg
aaaaaagatg tgaacagtga atcaatcaaa aattatcttt ataaaaactc tgatttacag
atttcatata atttcaacat ggtcgctatt agtgatggtc gtccaaaatt gatgggtatt
cgtcaaatta tagatagtta tttgaatcat caaattgagg ttgttgcaaa tagaacgaag
tttgaattag ataatgctga aaaacgtatg catatcgttg aaggtttgat taaagcgttg
tcaattttag ataaagtaat cgaattgatt cgtagctcta aaaacaagcg tgacgctaaa
gaaaacctta tcgaagtata cgagttcaca gaagaacagg ctgaagcaat tgtaatgtta
cagttatatc gtttaacaaa cactgacata gttgcgcttg aaggtgaaca taaagaactt
gaagcattaa tcaaacaatt acgtcatatt cttgataacc atgatgcatt attgaatgtc
ataaaagaag aattgaatga aattaaaaag aaattcaaat ctgaacgact gtctttaatt
gaagcagaaa ttgaagaaat taaaattgac aaagaagtta tggtgcctag tgaagaagtt
attttaagta tgacacgtca tggatatatt aaacgtactt ctattcgtag ctttaatgct
agcggtgttg aagatattgg tttaaaagat ggtgacagtt tacttaaaca tcaagaagta
aatacgcaag ataccgtact agtatttaca aataaaggtc gttatctatt tataccagtt
cataaattac gagatattcg ttggaaagaa ttggggcaac atgtatcaca aatagttcct
atcgaagaag atgaagtggt tattaatgtc tataatgaaa aggactttaa tactgatgca
ttttatgttt ttgcgactca aaatggcatg attaagaaaa gtacagtgcc tctatttaaa
acaacgcgtt ttaataaacc tttaattgca actaaagtta aagaaaatga tgatttgatt
agtgttatgc gctttgaaaa agatcaatta attaccgtaa ttacaaataa aggtatgtca
ttaacgtata atacaagtga actatcagat actggattaa gggcggctgg tgttaaatca
ataaatctta aagttgaaga tttcgttgtt atgacagaag gtgtttctga aaatgatact
atattgatgg ccacacaacg cggctcgtta aaacgtatta gttttaaaat cttacaagtt
gctaaaagag cacaacgtgg aataacttta ttaaaagaat taaagaaaaa tccacatcgt
atagtagctg cacatgtagt gacaggtgaa catagtcaat atacattata ttcaaaatca
aacgaagaac atggtttaat taatgatatt cataaatctg aacaatatac aaatggctca
ttcattgtag atacagatga ttttggtgaa gtaatagaca tgtatattag ctaaaaacta
tatgcaatca cgaaattaaa tgataaaata cagtaatgtt aaattttgac taaattcaag
ggatttatat taaatgctga ccaagtactt atcgttaaat tagcgatacg gaatccgcgg
aattc 4565

<210> 18
<211> 17
<212> DNA
<213> Artificial sequence
<220>
<223> M13 Primer M4
<400> 18
gttttcccagtcacgac 17

<210> 19
<211> 17
<212> DNA
<213> Artificial sequence
<220>
<223> M13 Primer RV
<400> 19
caggaaacagctatgac 17

## Description

### BACKGROUND OF THE INVENTION

### Field of the Invention

The present invention relates to a method of analyzing a nucleic acid.

### Description of the Related Art

Conventionally, a nucleic acid probe having a base sequence complementary with a specific base sequence is used to analyze the frequency of specific gene expressions in a sample nucleic acid (gene expression frequency analysis), and to analyze the increase/decrease of the copy number of a specific base sequence.

The gene expression frequency analysis is mainly performed by the double fluorescence labeling method using a DNA microarray (for example, refer to Non Patent Document 1). This analysis method is a system to observe the difference of gene expressions. That is, to detect the difference of gene expressions between mRNA samples obtained from two cell groups in different conditions.

First, at each time of analysis, mRNA samples from respective groups are labeled with different fluorescent labels and competitively hybridized on a DNA microarray having a solid-phased probe, and then fluorescence from respective groups are measured and compared. In this manner, whether the frequency of specific gene expression in one group is increased or decreased with respect to that in the other group, can be estimated.

Since a signal reflecting the result of competitive hybridization in a same spot is detected, this method is effective to examine a relative change of gene expression frequency.

On the other hand, a method of enabling a comprehensive detection of the increase/decrease of a specific base sequence present in a chromosome includes an Array Comparative Genomic Hybridization (ACGH) method (for example, refer to Patent Document 1).

In this method, an array having a large number of cloned DNA fragments and synthetic oligo DNAs spotted on a slide glass or the like, is made. Chromosome DNAs derived from a plurality of different cells are labeled with respectively different fluorescent dyes (such as Cy3 and Cy5), and competitively hybridized in the array. Based on the intensity ratio of the obtained fluorescent signals, a change between cells such as an increase/decrease of copy number and deletion of DNA in the area corresponding to the arrayed DNA fragment, can be detected. At this time, any change can be detected at one copy level.

Research aimed at cancer diagnosis is performed by using the above analysis methods such as the gene expression frequency analysis or ACGH method, to detect a difference of nucleic acid amount between a cancer cell and a normal cell.

If in practice a clinical specimen is analyzed by a microarray, the nucleic acid amount of the clinical specimen used for the analysis is often small. In this case, the nucleic acid of the clinical specimen is amplified by the Eberwine RNA amplification method or WGA method, and then analyzed.
Patent Document 1: Published Japanese Translation No. H11-510681 of PCT International Publication
Non Patent Document 1: Zhi-Jun Tan et. al., "World Journal of Gastroenterology", 2003, Vol. 9, p. 818-823

In the conventional ACGH method, one type of solid-phased probe is prepared with respect to one target base sequence, and by analyzing the presence/absence of hybridization therebetween, a change of DNA copy number in the area corresponding to the arrayed DNA fragment can be detected. However, the change of the primary structure in the molecule of a sample nucleic acid can not be analyzed.

In order to have an accurate value of the result obtained in the gene expression frequency analysis or ACGH method by means of a conventional microarray, a cell (such as a normal cell) used as a standard in the analysis is required to have a fixed amount of nucleic acid at all times. However, in practice, the nucleic acid amount varies due to the sampling method, culturing conditions, or the like, and it is difficult to obtain accurate comparison analytic values with high reproducibility. Moreover, if the nucleic acid amount supplied to the competitive hybridization is standardized by adjusting the nucleic acid amount derived from different cells at a fixed level, the nucleic acid amount may vary at each time of analysis or by each sample type, due to this adjustment itself.

Furthermore, in order to minimize the difference of uptaking efficiency of fluorescent labels into nucleic acids between cells to be compared, the same conditions except for the type of fluorescent label are set between these cells, to control the variance of the comparison analytic value caused by the difference of uptaking efficiency of the fluorescent labels. However, if a clinical sample is used in practice, even if the nucleic acid amount is adjusted, the extracted nucleic acid sample may differ in the quality due to contamination of proteins, polysaccharides, or the like. As a result, the uptaking efficiency of the fluorescent labels differs between samples to be compared, making the analysis results largely vary.

Moreover, if the nucleic acid amount of the clinical specimen used for the analysis is small, the nucleic acid of the clinical specimen is amplified. However, in particular, due to differences in the nucleic acid amount and the quality, the amplified amount differs between chromosome DNAs to be compared and analyzed, and the analysis can not be accurately performed.

Furthermore, a competitive hybridization is always required between two cells. Therefore, at each time of analysis, chromosome DNAs have to be extracted from the respective cells, and labeling reactions have to be performed with respect to the extracted chromosome DNAs. The labeling takes time and cost.

In the production of a DNA microarray used for analysis, the uniformity of size and shape of obtained probe spots is limited due to differences in batches of pin points. As a result, no sufficient reproducibility can be obtained in the analysis result utilizing hybridization. Moreover, between spots with solid-phased probes having different specificities from each other, signal values can not be simply compared for use in determination.

In order to quantify the nucleic acid by such a microarray, when an array is produced, spotting has to be done so that the solid-phased amounts of synthetic oligo DNA or cDNA used as a solid-phased probe nucleic acid are always at a fixed level within a certain range, in all chips to be produced. If it can not be produced so that the solid-phased amounts thereof are at a fixed level, there is a possibility of error caused by the variance of the solid-phased amount.

The present invention takes the above problems into consideration, with the object of avoiding any possibility of error caused by a nucleic acid used as a standard, a probe, or probe spot, etc. or enabling correction thereof, to analyze the frequency of gene expressions with high reproducibility, and to analyze any change of primary structure in a sample nucleic acid.

### SUMMARY OF THE INVENTION

The method of analyzing nucleic acid of the present invention is a method of analyzing a primary structure of at least one target base sequence present in a sample nucleic acid, by detecting any signal from a sample nucleic acid hybrid composed of the sample nucleic acid and a probe, wherein
any signal from a synthetic standard nucleic acid hybrid composed of the probe and a synthetic standard nucleic acid containing at least one target base sequence is detected and on the basis of the signal from the synthetic standard nucleic acid hybrid, the signal from the sample nucleic acid hybrid is corrected to thereby effect analysis of the primary structure of the sample nucleic acid.

In the analysis of the primary structure of the sample nucleic acid of the present invention, preferably the synthetic standard nucleic acid is previously pooled, so as to perform the analysis using the synthetic standard nucleic acid from the same pool.

Moreover, the synthetic nucleic acid is preferably synthesized from a chromosome extracted from a control cell.

Furthermore, the synthetic nucleic acid is preferably synthesized based on an already known base sequence.

The synthetic nucleic acid may contain a plurality of types of target base sequences in one molecule.

In the method of analyzing sample nucleic acid of the present invention, preferably the concentration and the quality of the pooled synthetic standard nucleic acid are previously measured.

Here, preferably the concentration and the quality of the synthetic standard nucleic acid, and the signal value specific to the synthetic standard nucleic acid are registered in a database, and the database is used for correction of a signal specific to the sample nucleic acid and/or comparison of signals between a plurality of sample nucleic acids.

Furthermore, the database is preferably set according to one or more classifications selected from an analysis condition, a base sequence of a probe, and a combination of the condition and the base sequence.

The method of the present invention uses a sample nucleic acid applied with a first labeling treatment, the synthetic standard nucleic acid applied with a second labeling treatment that is different from the first labeling treatment, and the probe applied with a third labeling treatment that is different from the first labeling treatment and the second labeling treatment, or which is solid-phased in the carrier surface, and comprises:
a first process for bringing the sample nucleic acid into contact with the probe to form a first hybrid, so as to measure a first signal intensity specific to the first hybrid;
a second process for bringing a fixed amount of the synthetic standard nucleic acid into contact with the probe, to form a second hybrid, so as to measure a second signal intensity specific to the second hybrid; and
a third process for correcting the first signal intensity on the basis of the second signal intensity.

The carrier is preferably a microarray.

Here, the first hybrid and the second hybrid are preferably formed on a same microarray.

Preferably, a process for correcting a variance between the first signal intensity and the second signal intensity is further included.

In the third process, preferably, a variance of the first signal intensity caused by an amount of the solid-phased probe is corrected on the basis of the second signal intensity.

Moreover, the first process and the second process are preferably performed by a kinetic assay.

The kinetic assay is preferably performed by changing one or more conditions selected from the temperature, pH, and composition of a reaction solution.

In the first labeling treatment and the second labeling treatment, a fluorescent dye is preferably used.

Moreover, using a plurality of types of the aforementioned probe; the first hybrid and the second hybrid with respect to a same probe may be formed separately with respect to the respective types of probes, and there may be further included a process of measuring a bonding amount ratio of the synthetic standard nucleic acid and the sample nucleic acid with respect to various probes, and a process of confirming the combination of the bonding amount ratio in the total probes.

Here, the synthetic standard nucleic acid preferably includes a plurality of target base sequences.

The method of the present invention is also suitable for a plurality of types of sample nucleic acids.

The nucleic acid analysis kit of the present invention is used in the method of analyzing nucleic acid of the present invention.

According to the present invention, it becomes possible to avoid any possibility of error caused by a nucleic acid used as a standard, a probe, or probe spot, etc., or to correct this. Consequently, the frequency of gene expressions can be analyzed with high reproducibility. Moreover, any change of primary structure in a sample nucleic acid can be analyzed.

### BRIEF DESCRIPTION OF THE DRAWINGS

FIG. 1 is a conceptual diagram showing a chromosome model in Example 1.
FIG. 2 is a plan view showing an arrangement of probes in an array used in Example 1.
FIG. 3 is a conceptual diagram showing a model of a target sample solution I in Example 1.
FIG. 4 is a conceptual diagram showing a model of a target sample solution II in Example 1.
FIG. 5 is a graph showing profiles (fluorescence intensity with respect to probe type) of a standard solution in Example 1.
FIG. 6 is a graph showing profiles (fluorescence intensity with respect to probe type) of the standard solution and the target sample I in Example 1, and an estimated primary structure.
FIG. 7 is a graph showing profiles (fluorescence intensity with respect to probe type) of the standard solution and the target sample II in Example 1, and an estimated primary structure.
FIG. 8 is a plan view showing an arrangement of probes in an array used in Example 2.

### DETAILED DESCRIPTION OF THE PREFERRED EMBODIMENTS

The method of analyzing nucleic acid of the present invention (hereunder, may be called "nucleic acid analysis method") is a method of analyzing the primary structure of a sample nucleic acid having a specific base sequence, wherein any signal from a synthetic standard nucleic acid hybrid composed of a probe and a synthetic standard nucleic acid containing at least one target base sequence is detected, and on the basis of the signal from the synthetic standard nucleic acid hybrid, the signal from the sample nucleic acid hybrid is corrected to thereby effect analysis of the primary structure of the sample nucleic acid.

In the present specification, the primary structure of a nucleic acid includes the presence/absence of a target base sequence in the nucleic acid molecule. It also includes the presence/absence of deletion, amplification, translocation, inversion, insertion, and the like of a specific region, compared with a normal chromosome or other samples.

In the method of the present invention, a signal from the synthetic standard nucleic acid hybrid is used as a basis at all times, that is, regardless of the number of times of analysis, type of an analysis device (such as a microarray), probe type, or the like.

In the method of the present invention, a synthetic nucleic acid which has been previously synthesized is used as a standard nucleic acid. It can be pooled and commonly preserved to use throughout analysis over a plurality of times, a plurality of devices, and a plurality of types of probes (such as a plurality of probe spots). The usage of the pooled synthetic standard nucleic acid enables to completely avoid any possibility of error caused by; the amount of the standard nucleic acid for comparative analysis, an adjustment operation of the nucleic acid amount, a variance of uptaking efficiency of fluorescent labels due to impurities, or the like.

Consequently, errors of signal intensity caused by factors other than due to standard nucleic acid, in the case where an array is used, such as the solid-phased amount of the probe, the size and shape of the spots, and the like, can be determined and corrected.

Therefore, if examinations are performed a plurality of times using the hybridization between a specific probe type and a sample nucleic acid to detect the signal intensity, the examination results over a plurality of times can correct each other. Consequently, firstly, the abundance (nucleic acid amount) of the target base sequence corresponding to the specific probe type can be measured with high reproducibility, and the gene expression frequency can be precisely analyzed. In the present specification, probe type means the differences of base sequences of the probe.

Moreover, by the following reason, secondly, not only the change of DNA copy number in the region corresponding to the arrayed DNA fragment can be analyzed, but also the primary structure in the sample nucleic acid can be identified, and the change of primary structure can be determined.

That is, in the conventional ACGH method using a cell extraction as a standard nucleic acid at each time of analysis, the following troubles occur if an attempt is made to analyze the change of primary structure of chromosome DNA derived from a target cell. When a standard nucleic acid and a sample nucleic acid are hybridized with a plurality of types of probes corresponding to the region whose primary structure is to be analyzed, since error factors exist in the standard nucleic acid side, there is no means to correct the errors due to the solid-phased amount of the respective probes, the size and shape of the spot, and the like, and hence the signal intensity of the hybrids corresponding to the respective probe types can not be quantitatively compared between the probe types.

On the other hand, in the present invention, since any possibility of error caused by the standard nucleic acid can be avoided, errors caused by factors from probes, probe spots, or the like, can be determined and corrected. Consequently, the signal intensity of the hybrids formed by the sample nucleic acid with respect to the respective types of probes, that is, the abundance of the target base sequence complementary with the respective types of probes in the sample nucleic acid, can be quantitatively compared. As a result, the change of primary structure of a nucleic acid sequence in the sample nucleic acid can be accurately determined.

Specifically, a probe type-signal intensity profile comprising a signal intensity with respect to the probe type can be formed as a reliable common one, regardless of the difference in spots having a solid-phased probe, the difference in microarrays, and the difference in number of times of hybridization.

According to the present invention, by using the synthetic standard nucleic acid as a basis, examination results corresponding to a plurality of types of sample nucleic acid-probe can be compared. Consequently, whether or not the primary structure differs between a plurality of types of sample nucleic acids can be reliably determined, demonstrating an advantageous effect compared to the conventional method.

Moreover, there is no need for an operation at each time of analysis to extract a nucleic acid from a cell for used for a standard, and to label it, so that the time and cost can be saved.

### (Sample nucleic acid)

As a sample nucleic acid to be analyzed by the nucleic acid analysis method of the present invention, chromosome DNA extracted from a specimen or the like may be supplied for the method of the present invention as it is, or the chromosome DNA may be supplied after being amplified. The amplification method is not specifically limited, and a publicly known method may be used.

### (Synthetic standard nucleic acid)

The synthetic standard nucleic acid used for the present invention comprises a synthetic nucleic acid that is previously synthesized, and includes at least one target base sequence, and is pooled.

Here, the target base sequence means a base sequence whose presence/absence is to be detected in the sample nucleic acid, in order to analyze the primary structure.

The synthetic standard nucleic acid may contain one type of target base sequence, or may contain a plurality of types of target base sequences.

If the synthetic standard nucleic acid contains a plurality of types of target base sequences, the synthetic standard nucleic acid may be a mixture having a plurality of types of synthetic nucleic acid molecules containing respectively different target base sequences, or may comprise a synthetic nucleic acid molecule containing a plurality of types of target base sequences in one molecule. However, it is preferable that the synthetic nucleic acid molecule contains a plurality of types of target base sequences in one molecule. By containing a plurality of types of target base sequences in one molecule, the synthetic standard nucleic acid used when the target base sequences are present in close regions on the chromosome can be made similar to the real sample. Therefore analysis error can be further reduced. Even if the target base sequences are not present in close regions on the chromosome, correction can be performed regarding various target base sequences with a few types of synthetic nucleic acids. Therefore it is advantageous from the point of reducing the types of synthetic nucleic acids to be prepared.

The synthetic nucleic acid can be synthesized by means of either an enzyme reaction or chemically. In the case of the enzyme reaction, the enzyme reaction may be the same as that of the amplification treatment of the sample nucleic acid, or may be different therefrom.

The synthetic nucleic acid is preferably synthesized from a chromosome extracted from a control cell.

Even if it is derived from a control cell, the common one is used at all times as the synthetic nucleic acid that has been previously synthesized and pooled, and thereby the possibility of error caused by the standard nucleic acid can be avoided. By using a synthetic nucleic acid derived from the control cell, if the sample nucleic acid is derived from the test group cell, the primary structure can be particularly readily compared between the control group and the test group.

### (Probe)

The probe used in the present invention has a base sequence that is complementary with the target base sequence contained in the synthetic standard nucleic acid. If there are a plurality of types of target base sequences to be detected in the sample nucleic acid, it is preferable to prepare respective probes that are complementary to the respective target base sequences.

If a fixed amount of pooled synthetic standard nucleic acid is used at all times (for example, regardless of the difference in the sample nucleic acid) in the present invention, the difference of the primary structure between a plurality of sample nucleic acids can be detected. Here, if a plurality of sample nucleic acids are derived from different test groups or different cells, the nucleic acid primary structure can be compared and determined between the test groups or the cells.

Furthermore, if the concentration and the quality of the pooled synthetic standard nucleic acid are previously measured, the primary structure of the specific sample nucleic acid can be estimated without requiring the comparison between a plurality of sample nucleic acids.

Examples of an index of the quality include; the type of base sequence contained in the whole synthetic standard nucleic acid, the rate of content of each base sequence, and the rate of content of impurities such as proteins or polysaccharides.

Preferably, the concentration and the quality of the synthetic standard nucleic acid, and the signal intensity value specific to the synthetic standard nucleic acid are registered in a database, and the database is used for correction of a signal specific to the sample nucleic acid and/or comparison of signals between a plurality of sample nucleic acids.

The database is preferably set according to one or more classifications selected from; the analysis condition, the base sequence of the probe, and the combination of the condition and the base sequence.

Examples of the analysis condition include; a condition of hybridization reaction, the type of labeling treatment with respect to the sample nucleic acid/synthetic standard nucleic acid, and a condition for detecting the signal intensity, as described later.

Hereunder embodiments of the present invention are exemplified.

In one embodiment, the present aspect uses a sample nucleic acid applied with a first labeling treatment, the synthetic standard nucleic acid applied with a second labeling treatment that is different from the first labeling treatment, and the probe applied with a third labeling treatment that is different from the first labeling treatment and the second labeling treatment, or which is solid-phased in the carrier surface, and comprises: a first process for bringing the sample nucleic acid into contact with the probe to form a first hybrid, so as to measure a first signal intensity specific to the first hybrid; a second process for bringing a fixed amount of the synthetic standard nucleic acid into contact with the probe, to form a second hybrid, so as to measure a second signal intensity specific to the second hybrid; and a third process for correcting the first signal intensity on the basis of the second signal intensity.

In this embodiment, the sample nucleic acid is previously applied with the first labeling treatment.

Moreover, the synthetic standard nucleic acid is applied with the second labeling treatment. The probe is applied with the third labeling treatment, or is solid-phased in the carrier surface. Consequently, the first hybrid formed from the probe and the sample nucleic acid, and the second hybrid formed from the probe and the synthetic nucleic acid can be detected corresponding to the probe type. These first, second, and third labeling treatments are not specifically limited as long as it is possible to separately detect in the respective detection step.

For example, various fluorescent substances can be used as labels. Besides various fluorescent dyes, there may be used fluorescent glass particles, fluorescent semiconductor particles, and the like.

Moreover, detection can be also performed by means of scattered light or reflected light, and metal particles or dielectric particles may be used as labels. For example, there may be used particles of gold, silver, platinum, and silicon, or latex particles. In particular, metal particles of gold, silver, platinum, or the like having a particle size of 10 to 100 nm are preferred, since the speed of the particles in the kinetic state becomes optimum. Moreover, latex particles having a particle size of 0.1 to 1 µm are preferred, since similarly the speed of the particles in the kinetic state becomes optimum. Here, examples of the kinetic state of particles include Brownian motion and vibration. A suitable particle size is appropriately determined by the specific gravity of the particles, the speed of Brownian motion, or the like.

It is possible to use substances forming a specific bond such as a reaction between biotin and avidin to label with the abovementioned fluorescent substance, metal particles, dielectric particles, or the like. Furthermore, it is also possible to use substances bonded with an enzyme forming the specific bond, and supply a substrate thereto, so as to perform detection using chemiluminescence or chemical reaction. It is also possible to label with a radioisotope.

Considering the easiness and the cost of labeling treatment and the easiness of detection, a fluorescent dye is preferably used for the labeling treatment.

### (First process and second process)

The first process and the second process may be performed one by one in any order, or may be performed at the same time.

In the first process, the sample nucleic acid is brought into contact with the probe. By so doing, the first hybrid composed of the probe and the sample nucleic acid is formed. Then, the first signal intensity specific to the first hybrid is measured.

In the second process, the synthetic standard nucleic acid is brought into contact with the probe. By so doing, the second hybrid composed of the probe and the synthetic nucleic acid is formed. Then, the second signal intensity specific to the second hybrid is measured.

At this time, the type of probe used for the first process and the second process is common to each other. The first hybrid and the second hybrid may be formed by bringing the sample nucleic acid and the synthetic standard nucleic acid into contact with the same probe at the same time in a competitive manner, or may be formed by bringing them into contact with the same probe one by one in a non competitive manner. Moreover, as long as the type of probe is common, the sample nucleic acid and the synthetic standard nucleic acid may be respectively brought into contact with different probe molecules.

The first process and the second process may be performed, for example by the following method.

Firstly, the probe is brought into contact with a reaction solution containing the synthetic standard nucleic acid and the sample nucleic acid, to effect the hybridization reaction. Here, the temperature, time, method, and the like of the hybridization reaction are not limited either. Moreover, the pH, salt concentration, and the like of the reaction solution are not limited.

Then, for example if the probe is solid-phased in the carrier surface, the signal intensity specific to the first labeling treatment and the signal intensity specific to the second labeling treatment are respectively measured for each probe spot.

### (Third process)

The first process and the second process are followed by the third process.

In the third process, the first signal intensity is corrected on the basis of the second signal intensity.

After the first process and the second process but before the third process, a process for removing the nucleic acid that has not formed a hybrid with the probe, may be performed as required. For example, if a probe which is solid-phased in the carrier surface is used, the carrier surface may be washed. Moreover, if a probe conjugated with magnetic beads or the like, is used the reaction solution comprising the hybrid and the unreacted nucleic acid can be applied with a magnetic field so as to separate the hybrid containing the magnetic beads from the unreacted nucleic acid not containing the magnetic beads. If this method is used, reaction and measurement can be performed in a liquid phase, without going through a reaction in a solid phase.

In this embodiment, the synthetic standard nucleic acid is previously synthesized and pooled. The synthetic standard nucleic acid is always used as the basis since the first signal intensity is corrected on the basis of the second signal intensity specific to the second hybrid containing the synthetic standard nucleic acid.

Here, either one type of probe or a plurality of types of probes may be used. Particularly, the method of using a plurality of types of probes is suitable in order to determine the change of the primary structure in a specific region of the sample nucleic acid. The method suitable for the analysis of the primary structure in a specific region is described later in detail.

If a probe which is solid-phased in a carrier is used, in the third process, the variance of the first signal intensity caused by the amount of the solid-phased probe can be corrected on the basis of the second signal intensity.

For example, by using the synthetic standard nucleic acid regardless of the number of times of analysis, the variance of the first signal intensity caused by the variance of the amount of the solid-phased probe at each time of analysis if the specific probe is focused on, can be corrected. Therefore, the absolute or relative abundance of the target base sequence in the sample nucleic acid can be obtained with high reproducibility.

Moreover, using a plurality of types of probes and the synthetic standard nucleic acid regardless of the type of probe, the variance of the first signal intensity caused by the variance of the amount of the solid-phased probe between probe types if the sample nucleic acid is focused on, can be corrected. Therefore, the first signal intensities specific to the respective probe types can be quantitatively compared with each other.

If one type of sample nucleic acid is analyzed, preferably, using a fixed amount of the synthetic standard nucleic acid whose quality is clarified, the first signal intensity is corrected on the basis of the second signal intensity, and thereby the abundance of the target base sequence in the sample nucleic acid can be quantified with high reproducibility. Furthermore, if the synthetic nucleic acid is synthesized based on an already known base sequence, and the rate of content of the target base sequence in the synthetic standard nucleic acid has a composition corresponding to an already known normal type or a mutant type, whether the sample nucleic acid is the normal type or a mutant type can be readily determined.

If a plurality of types of sample nucleic acids are analyzed, the first process and the second process are separately performed for the respective sample nucleic acids, so as to obtain a plurality of first signals corresponding to the respective sample nucleic acids. In this case, the quality of the synthetic standard nucleic acid need not be measured in advance.

By using a fixed amount of the pooled synthetic standard nucleic acid regardless of the difference of the sample nucleic acid, the respective first signal intensities with respect to the same type of probe can be corrected on the basis of the second signal intensity, and can be compared with each other. Consequently, the primary structure can be accurately compared between sample nucleic acids derived from separate sites in the same body, derived from the same site in the same body but sampled from a specimen at different times, derived from a control group and a test group, and the like. For example, whether the target base sequence in one sample nucleic acid is increased or decreased with respect to the other sample nucleic acid, can be accurately determined.

In this embodiment, if the probe is solid-phased in a carrier surface, various types of carrier may be employed. For example, a two-dimensional substrate such as a silicon wafer or a glass, a membrane filter, a microtiter plate, various glass beads and resin beads, various porous substrates, and various gels can be employed. Here, a microarray is preferably used. In the carrier such as a microarray, a plurality of types of probe nucleic acids may be solid-phased either in the same spot or different spots.

At this time, the first hybrid and the second hybrid are preferably formed on the same microarray. Furthermore, the first hybrid and the second hybrid are preferably formed in a competitive manner with respect to the same probe.

In this embodiment, preferably a process for correcting the variance between the first signal intensity and the second signal intensity is further included. This is advantageous for correcting the difference of hybridization efficiency due to difference between the sample nucleic acid and the synthetic standard nucleic acid, so as to analyze the primary structure of the nucleic acid more accurately.

The first process and the second process are preferably performed by a kinetic assay.

As the kinetic assay, for example, a plurality of the above hybridization conditions may be set and the data of signal intensity value may be kinetically acquired.

Kinetic data acquisition means to perform data acquisition while changing measurement conditions and detection conditions of the hybrid formation reaction by means of complementarity between the target base sequence and the probe. The kinetic data acquisition is not performed at a fixed time point, but for example, while changing one or more conditions selected from the temperature, pH, and composition of the reaction solution, for a measurement time within a range between several minutes and several hours. The change of the reaction condition can be performed either in a stepwise manner or continuously.

The hybrid is normally formed in an estimated optimum condition based on the sequence data. Consequently, it is usually unknown whether or not the condition is really optimum. As a result, if a measurement is performed in a static (non kinetic) manner or fixed pointedly, there is a possibility that the data acquisition is performed under a non optimum condition.

On the other hand, if the first process and the second process are performed by a kinetic assay to perform the kinetic data acquisition, the hybridization reaction is promoted in a plurality of reaction conditions, and can be measured with time. Consequently, even if a plurality of types of probes having different characteristic values peculiar to the sequence, such as Tm value, are solid-phased in the same assay, kinetic data acquisition makes it possible to experiment in consideration of the range of the characteristic value, so as to accurately detect a plurality of types of sequences almost at the same time.

As described above, the method of using a plurality of types of probes is particularly suitable in order to determine the change of primary structure in a specific region of the sample nucleic acid. The primary structure in a specific region can be estimated, for example by using a plurality of types of the aforementioned probe; forming the first hybrid and the second hybrid with respect to the same probe separately for the respective types of probes; and further including a process of measuring the bonding amount ratio of the synthetic standard nucleic acid and the sample nucleic acid with respect to various probes, and a process of confirming the combination of the bonding amount ratio in the total probes.

In order to measure the bonding amount ratio, there may be used the sample nucleic acid applied with the first labeling treatment, the synthetic standard nucleic acid applied with the second labeling treatment that is different from the first labeling treatment, and a probe applied with a third labeling treatment that is different from the first labeling treatment and the second labeling treatment, or which is solid-phased in the carrier surface.

Using this method, whether the primary structure of the sample nucleic acid is a normal type or a mutant type, and/or whether or not the primary structure differs between a plurality of sample nucleic acids, can be readily determined.

Examples of mutation include deletion, amplification, translocation, inversion, and insertion of the base sequence in a specific region.

This method can be performed for example by the following procedure.

Firstly, the synthetic standard nucleic acid and the sample nucleic acid are hybridized with the probe, so as to form the first hybrid and the second hybrid. However, in this method, the first hybrid and the second hybrid are necessarily formed in a competitive manner or a non competitive manner with respect to the same probe.

The first signal intensity specific to the first hybrid is measured by each probe type. Moreover, a standard profile comprising the signal intensity corresponding to the respective probe types is formed. The second signal intensity specific to the second hybrid is measured for the same probe. Moreover, a target profile comprising the signal intensity corresponding to the respective probe types is formed.

A combination of the respective binding amount ratios of the various probes can be indirectly confirmed as a combination of the first and the second signal intensity ratios of the various probes, over a plurality of probes.

If the synthetic standard nucleic acid has a structure that is previously clarified as a normal type or a mutant type, then by evaluating the bonding amount ratio of the respective probes using the standard profile and the target profile, it can be determined whether a single sample nucleic acid is a normal type or a mutant type. Moreover, examination is performed using the synthetic standard nucleic acid with respect to a plurality of sample nucleic acids commonly regardless of the composition. By comparing the examination results with each other, the difference of primary structure between the sample nucleic acids can be detected.

In the method of using a plurality of types of probes, a synthetic standard nucleic acid containing a plurality of types of target base sequences is preferably used.

The nucleic acid analysis kit of the present invention is used in the method of analyzing nucleic acid of the present invention described above.

This nucleic acid analysis kit includes: a synthetic standard nucleic acid which comprises a synthetic nucleic acid that is previously synthesized, and includes at least one target base sequence, and is pooled; and a probe having a base sequence that is complementary with the target base sequence.

Preferably, the synthetic standard nucleic acid is applied with a labeling treatment, the probe is applied with a labeling treatment that is different from the synthetic standard nucleic acid, or is solid-phased in a carrier surface.

Moreover, as required, there may be a plurality of types of target base sequences contained in the synthetic standard nucleic acid, or there may be a plurality of types of probes.

Shown next shown a model of a method of determining the presence/absence of a change of the primary structure of the chromosome (with respect to the primary structure of a normal type), that was performed based on the present invention.

### Example 1

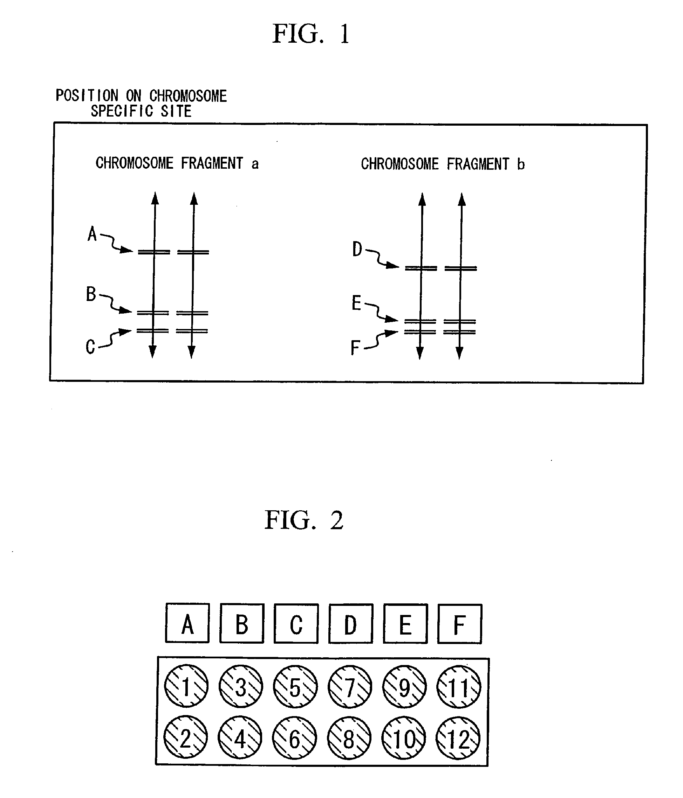

The specific regions of A, B, C, D, E, and F on the chromosomes as shown in FIG. 1 were assumed. It was assumed that the regions A, B, and C were on the same chromosome "a" and the regions D, E, and F were on another chromosome "b". The change of the primary structure due to deletion or amplification of these specific regions on the chromosomes, was analyzed. The base sequences corresponding to the regions A, B, C, D, E, and F are the target base sequences in the present example.

Hereunder, an experiment was performed using oligo DNAs corresponding to the regions A, B, C, D, E, and F.

The experiment step comprises 1) production of a microarray, 2) production of a fluorescent standard sample (synthetic standard nucleic acid), 3) production of a fluorescent target sample (sample nucleic acid), 4) hybridization of the standard sample and the target sample with respect to the microarray, and 5) data analysis.

A chemically synthesized oligo DNA was used as a sample. A probe (sequence number 1-2) for detecting a base sequence corresponding to A, a probe (sequence number 3-4) for detecting a base sequence corresponding to B, a probe (sequence number 5-6) for detecting a base sequence corresponding to C, a probe (sequence number 7-8) for detecting a base sequence corresponding to D, a probe (sequence number 9-10) for detecting a base sequence corresponding to E, and a probe (sequence number 11-12) for detecting a base sequence corresponding to F were solid-phased on a porous substrate in the arrangement of FIG. 2. In FIG. 2, reference symbols A to F correspond to regions A to F shown in FIG. 1, and numbers 1 to 12 correspond to sequence Nos. 1 to 12.

Oligo DNAs which are base sequences complementary with the respective probe sequences and have the 5' terminal labeled with Cy5 labels, were chemically synthesized. A standard solution (synthetic standard nucleic acid solution) containing 50 nM of each synthetic oligo DNA and having pH 7.5 adjusted by 10 mM Tris-HCl was prepared.

Moreover, oligo DNAs which are base sequences complementary with the respective probe sequences and have the 5' terminal labeled with Cy3 labels, were chemically synthesized. A target sample solution I containing 50 nM of each region A, B, C, D, E, or F and having pH 7.5 adjusted by 10 mM Tris-HCl was prepared.

Furthermore, a target sample solution II containing 25 nM of each region A or D or 50 nM of each region B, C, E, or F, and having pH 7.5 adjusted by 10 mM Tris-HCl was prepared.

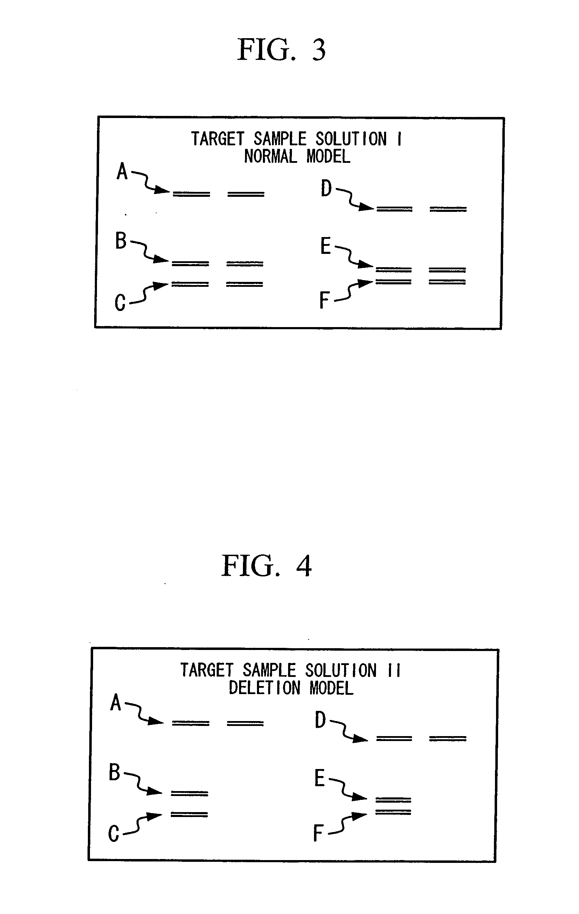

That is, as shown in FIG. 3, the target sample solution I is a model having no change in the chromosome structure in the A, B, and C regions and the D, E, and F regions, whose DNA amount is normal. As shown in FIG. 4, the target sample solution II is a model having normal A and D regions, but having LOH (elimination of heterojunction) occurring in the B, C, E, and F regions on the chromosomes, whose DNA amount is half of normal.

The hybridization was analyzed by a PAM microarray system: FD 10 manufactured by Olympus Corporation. This experimental system was designed to automatically drive the solution around the reaction filter and control the temperature thereof, and to record the fluorescent spot image. To the reaction site of a single-purpose chamber set with a microarray having a diameter of 6 mm, was added the standard solution and the target sample solution mixed with a hybridization buffer in the following way. Then, the solution was driven, the temperature was changed, and the fluorescent image was captured.

Firstly, in the verification of the model having no change in the chromosome structure, whose DNA amount was normal, 10 µl of the standard solution and 10 µl of the target sample solution I were dissolved in a hybridization buffer to make 3 x SSPE. This was added on a microarray, which was then taken into/taken out from a porous nucleic acid reaction carrier for 30 times at 50°C (drive control).

Moreover, in another array, in the verification of the model having LOH occurring on the chromosomes, whose DNA amount was half of normal, 10 µl of the standard solution and 10 µl of the target sample solution II were dissolved in a hybridization buffer to make 3 x SSPE. This was added on a microarray, which was then taken into/taken out from a porous nucleic acid reaction carrier for 30 times at 50°C (drive control).

In these reactions, the standard solution and each target sample solution were hybridized with the solid-phased probes for detecting the A to F base sequences, to form the first hybrids and the second hybrids.

Next, signals of the respective fluorescent dyes of Cy3 and Cy5 in each spot were detected, and the signal intensity data was acquired. The obtained image was analyzed using an analysis software that had been programmed for analyzing fluorescence intensity. By observing the intensity of fluorescence of two colors in one spot, the abundance of the sequence could be estimated.

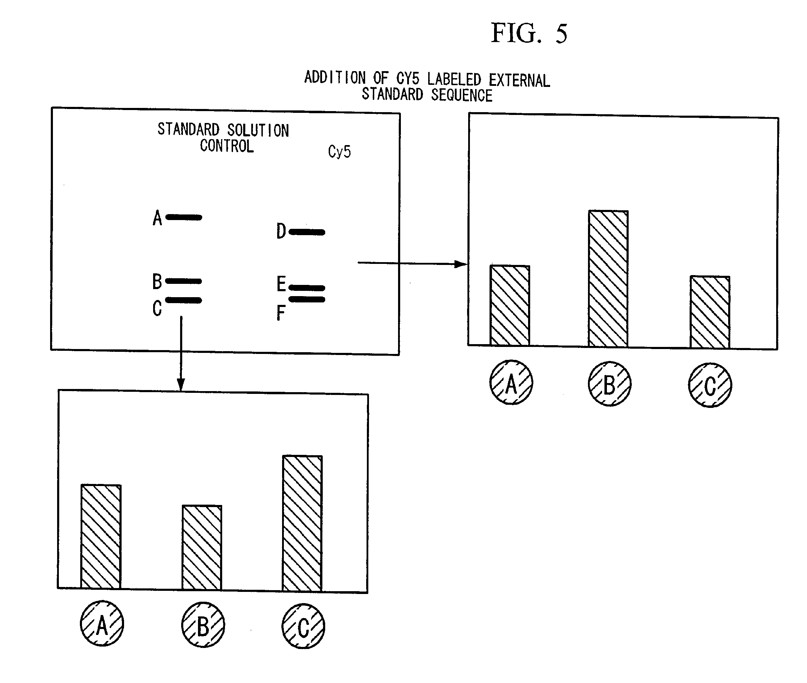

Hereunder, in FIGS. 5 to 7, the horizontal axis of the bar graph shows probe types (probes respectively complementary with A to F) and the vertical axis shows the fluorescence intensity.

That is, regarding the standard solution, Cy5 fluorescence in the respective spots was shown as in FIG. 5. The hybridization efficiency with respect to the respective solid-phased probes varies depending on the higher-order structure. However, the respective oligo DNA amount in the standard solution was previously set, and the fluorescence intensity of each spot serving as a basis could be measured. The fluorescence intensity profiles of the respective spots hybridized in two arrays showed similar forms.

Moreover, the variance of the solid-phased amount in the spots between two microarrays used at this time was very small, and assumed to be within a permissible range.

Furthermore, since this standard solution was pooled, when the experimental result did not match with the expectation later on, the history of the standard sample could be confirmed.

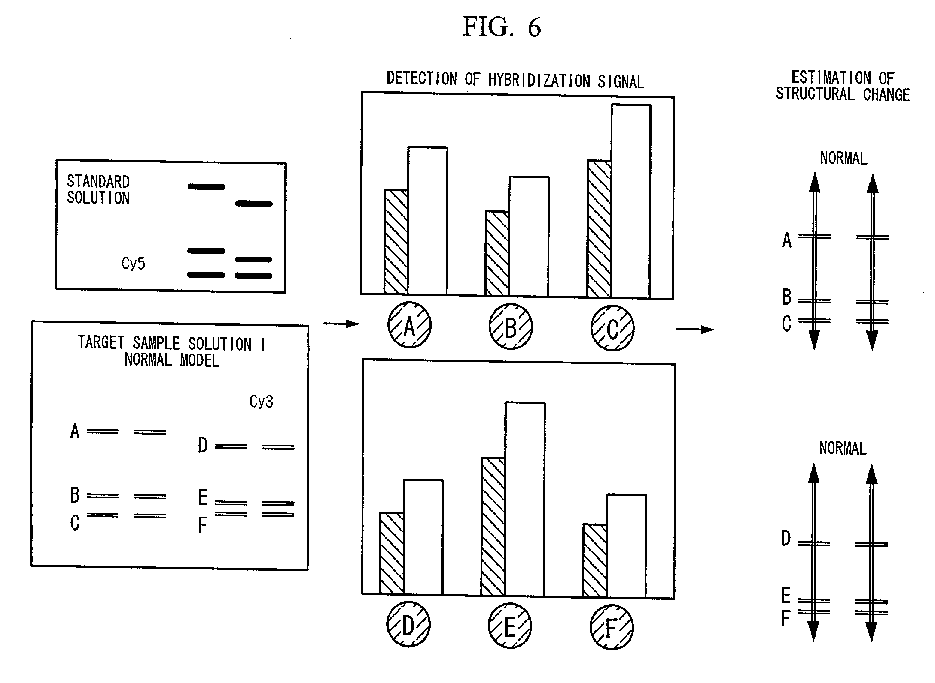

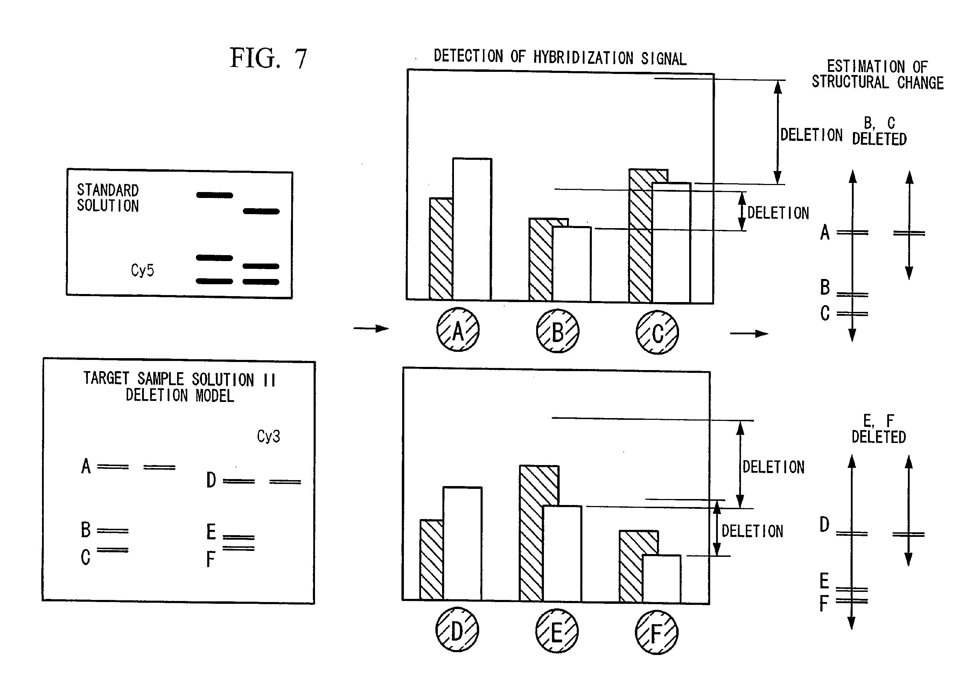

Next, the hybridization signal of the Cy3 labeled target sample solution was analyzed. In FIG. 6 and FIG. 7, the bar graph shown by oblique lines is the same bar graph as shown in FIG. 5 which shows the fluorescence intensity by examination of the standard solution.

As shown in FIG. 6, in the target sample solution I, there is a difference from the fluorescence intensity using the standard solution (A to C are about 1.3 times the signal of the standard solution, and D to F are about 1.4 times the signal of the standard solution), however A to F show similar profiles to those of the standard solution, and it can be assumed that the chromosome structure is not changed and the DNA amount is normal.

As shown in FIG. 7, in the target sample solution II, A and B show similar profiles to those of the standard solution (A is about 1.3 times the signal of the standard solution, and D is about 1.4 times the signal of the standard solution), however the fluorescent amounts of the B, C, E, and F regions were decreased to 0.65 times (B and C) to 0.7 times (E and F) the signal of the standard solution, and it can be assumed that the DNA amount was decreased. In this manner, in the target sample solution II, it can be assumed that the A and D regions are normal whereas LOH occurred in the B, C, E, and F regions on the chromosomes, and the DNA amount was decreased with respect to the target sample solution I serving as a normal model.

### Example 2

A model for analyzing a deletion in a certain PCR product is shown.

As a sample, a plasmid pUC 18 was cut by a restriction enzyme EcoRI (TaKaRa), and a sequence No. 17 was inserted thereinto by ligation, so as to prepare a sample I. Moreover, another plasmid pUC 18 was cut by AccI, and 515bp obtained by cutting the sequence No. 17 by the restriction enzyme AccI (DNA fragment cut from 286th base to 801 st base of sequence No. 17) was inserted thereinto.

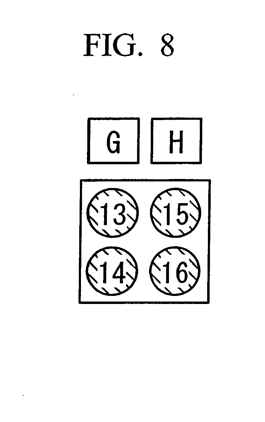

A sequence No. 13 being a sequence G of 35bp from 356th base of sequence No. 17, a sequence No. 14 being a complementary chain thereof, a sequence No. 15 being a sequence H of 37bp from 2550th base, and a sequence No. 16 being a complementary chain thereof were solid-phased on a microarray comprising a porous substrate, in the arrangement shown in FIG. 8.

Oligo DNAs which are base sequences complementary with the respective probe sequences and have the 5' terminal labeled with Cy5 labels were chemically synthesized. A standard solution containing 50 nM of each oligo DNA and having pH 7.5 adjusted by 10 mM Tris-HCl was prepared.

Moreover, using a M 13 Primer M 4 of a sequence No. 18 and a M 13 Primer RV of a sequence No. 19 having the 5' terminal labeled with Cy3, amplification was performed on the plasmid of the sample I as a template by PCR. Moreover, in the same manner, amplification was performed using a sample II as a template.

That is, the sample I is a sample nucleic acid including the whole sequence No. 17 and has the sequence G and the sequence H. On the other hand, the sample II has 515bp being a part of the target base sequence, and the sequence G, but does not have the sequence H.

There were prepared a sample (normal model sample) using 10 µl of sample I PCR product only, and a sample (deletion model sample: deletion occurs in PCR product and the region H was decreased to 1/2) using a mixture of 5 µl of sample I PCR product and 5 µl of sample II PCR product.

The hybridization was analyzed by a PAM microarray system: FD 10 manufactured by Olympus Corporation. This experimental system was designed to automatically drive the solution around the reaction filter and control the temperature thereof, and to record the fluorescent spot image. To the reaction site of a single-purpose chamber set with a microarray having a diameter of 6 mm, was added the standard solution and the target sample solution mixed with a hybridization buffer. Then, the solution was driven, the temperature was changed, and the fluorescent image was captured.

Firstly, in the verification of the model having no change in the chromosome structure, whose DNA amount was normal, 10 µl of the standard solution and 10 µl of the target sample solution I were dissolved in a hybridization buffer to make 3 x SSPE. This was added on a microarray, which was then taken into/taken out from a porous nucleic acid reaction carrier for 30 times at 50°C (drive control).

Moreover, in another array, in the verification of the deletion model, 10 µl of the standard solution and 10 µl of the deletion model sample were dissolved in a hybridization buffer to make 3 x SSPE. This was added on a microarray, which was then taken into/taken out from a porous nucleic acid reaction carrier for 30 times at 50°C (drive control).

In these reactions, the standard solution and each model sample were hybridized with the solid-phased probes for detecting the G and H base sequences, to form the first hybrids and the second hybrids.

Next, signals of the respective fluorescent dyes of Cy3 and Cy5 in each spot were detected. The obtained image was analyzed using an analysis software that had been programmed for analyzing fluorescence intensity, and the abundance of the sequence was estimated. As a result, by observing the intensity of fluorescent of two colors in one spot, the abundance of the sequence could be estimated.

That is, regarding the standard solution, Cy5 fluorescence in respective spots was shown. The fluorescence intensity of each spot serving as a basis could be measured, and the fluorescence intensity profiles of the respective hybridized spots in two arrays showed similar forms.

Next, the hybridization signal of the Cy3 labeled normal model sample was analyzed. The probes for detecting regions G and H show similar profiles to those of the standard solution (the fluorescent intensity ratio of G is about twice the standard solution, and H is about 1.5 times thereof), and it can be assumed that the structure in the sample nucleic acid is not changed and the DNA amount is normal. In the deletion model sample, the region G showed a similar profile to that of the standard solution, however the fluorescent amount of the region H was decreased to 0.67 times the standard solution, and it can be assumed that the DNA amount was decreased. In this manner, structural change due to a deletion in a molecule amplified by a PCR product could be estimated.

## Claims

1. A method for analyzing a primary structure of at least one target base sequence present in a sample nucleic acid, by detecting any signal from a sample nucleic acid hybrid composed of the sample nucleic acid and a probe, wherein
any signal from a synthetic standard nucleic acid hybrid composed of the probe and a synthetic standard nucleic acid containing at least one target base sequence is detected and on the basis of the signal from the synthetic standard nucleic acid hybrid, the signal from the sample nucleic acid hybrid is corrected to effect analysis of the primary structure of the sample nucleic acid.

2. A method for analyzing nucleic acid according to claim 1, wherein said synthetic standard nucleic acid is previously pooled, so as to perform the analysis using the synthetic standard nucleic acid from the same pool.

3. A method for analyzing nucleic acid according to claim 1 or 2, wherein said synthetic nucleic acid is synthesized from a chromosome extracted from a control cell.

4. A method for analyzing nucleic acid according to any one of claims 1 to 3, wherein said synthetic nucleic acid is synthesized based on an already known base sequence.

5. A method for analyzing nucleic acid according to any one of claims 1 to 4, wherein said synthetic nucleic acid contains a plurality of types of target base sequences in one molecule.

6. A method of analyzing nucleic acid according to any one of claims 2 to 5, wherein a concentration and a quality of said pooled synthetic standard nucleic acid are previously measured.

7. A method of analyzing nucleic acid according to claim 6, wherein the concentration and the quality of said synthetic standard nucleic acid, and the signal value specific to the synthetic standard nucleic acid are registered in a database, and the database is used for correction of a signal specific to the sample nucleic acid and/or comparison of signals between a plurality of sample nucleic acids.

8. A method of analyzing nucleic acid according to claim 7, wherein said database is set according to one or more classifications selected from an analysis condition, a base sequence of a probe, and a combination of said condition and said base sequence.

9. A method of analyzing nucleic acid according to any one of claims 1 to 7, using a sample nucleic acid applied with a first labeling treatment, said synthetic standard nucleic acid applied with a second labeling treatment that is different from the first labeling treatment, and said probe applied with a third labeling treatment that is different from the first labeling treatment and the second labeling treatment, or which is solid-phased in the carrier surface, and comprising:
a first process for bringing said sample nucleic acid into contact with said probe to form a first hybrid, so as to measure a first signal intensity specific to the first hybrid;
a second process for bringing said synthetic standard nucleic acid into contact with said probe, to form a second hybrid, so as to measure a second signal intensity specific to the second hybrid; and
a third process for correcting said first signal intensity on the basis of said second signal intensity.

10. A method of analyzing nucleic acid according to claim 9, wherein said carrier is a microarray.

11. A method of analyzing nucleic acid according to claim 10, wherein said first hybrid and said second hybrid are formed on the same microarray.

12. A method of analyzing nucleic acid according to any one of claims 9 to 11, wherein a process for correcting a variance between the first signal intensity and the second signal intensity is further included.

13. A method of analyzing nucleic acid according to any one of claims 9 to 12, wherein in said third process, a variance of the first signal intensity caused by an amount of the solid-phased probe is corrected on the basis of said second signal intensity.

14. A method of analyzing nucleic acid according to any one of claims 9 to 13, wherein said first process and said second process are performed by a kinetic assay.

15. A method of analyzing nucleic acid according to any one of claims 9 to 13, wherein said kinetic assay is performed by changing one or more conditions selected from the temperature, pH, and composition of a reaction solution.

16. A method of analyzing nucleic acid according to any one of claims 9 to 13, wherein a fluorescent dye is used in the first labeling treatment and the second labeling treatment.

17. A method of analyzing nucleic acid according to any one of claims 9 to 16 further comprising:
using a plurality of types of said probes;
forming said first hybrid and said second hybrid with respect to a same probe separately with respect to the respective types of probes; and
further including a process of measuring a bonding amount ratio of the synthetic standard nucleic acid and the sample nucleic acid with respect to various probes, and
a process of confirming the combination of the bonding amount ratio in the total probes.

18. A method of analyzing nucleic acid according to claim 17, wherein said synthetic standard nucleic acid includes a plurality of target base sequences.

19. A method of analyzing nucleic acid according to any one of claims 1 to 18, performed for a plurality of types of sample nucleic acids.

20. A nucleic acid analysis kit used in the method of analyzing nucleic acid according to any one of claims 1 to 19.
